# EUROPEAN PATENT APPLICATION

(11) **EP 0 790 493 A1**
(43) Date of publication of application: **20.08.1997**
(21) Application number: 97830049.9
(22) Date of filing: 11.02.1997
(51) Int. Cl.: G01K 13/00, A61B 5/00

(54) **Device for preventing convulsive attacks**

(30) Priority: 13.02.1996 IT TO960097
(71) Applicant: Dattoli, Luigi, 10024 Moncalieri (TO) (IT)
(72) Inventor: Dattoli, Luigi, 10024 Moncalieri (TO) (IT)

(57) **Abstract**

The present invention regards a device for preventing convulsive attacks.

Items Nos. 3 and 4, which are shown enlarged in the drawing of Fig. 2, represent the "heart" of this device. These items contain a temperature sensor which, via an analogue-to-digital converter, is able to detect the body temperature of the subject. The temperature detected appears on a liquid-crystal display and is constantly configured according to a threshold set by the user by means of a selector.

When the threshold set is overstepped, an alarm signal is activated.

The device is supplied by a battery, and the ON/OFF switch acts directly on the power supply circuit.

This device may be worn by means of a harness made of an elasticated, ecological material, with a hooking system, as shown in items Nos. 5 and 6 of the above-mentioned figure.

## Description

The present invention regards a device for preventing convulsive attacks.

I have had the opportunity to observe the variuos ways in which convulsions manifest themselves in small children, and in particular I have noted that each subject has his own level of body temperature above which convulsive seizures are triggered.
If this threshold value could be detected in some way, a timely intervention could prevent children with problems of this sort from having such attacks.

The major difficulty is encountered during night-time, when the parents are sleeping and the child's temperatures may rise abruptly.

Consequently, I have asked myself the question whether it would be possibile to create a device such as to enable timely intervention at the moment when the child's temperature overstepped the danger threshold by administering antipyretics or adopting other adequate preventive treatment.

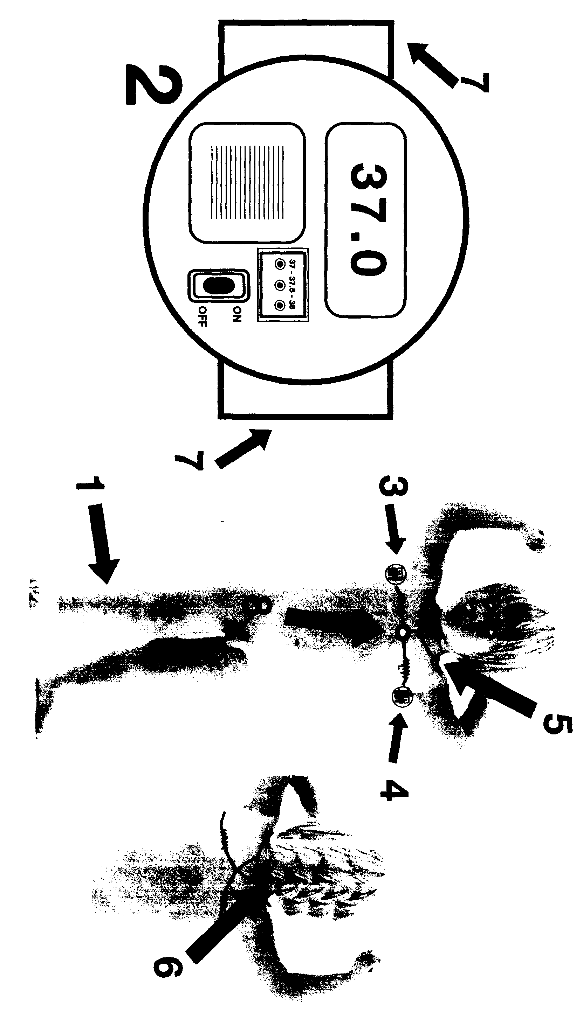

The following is a description of my intervention: Fig. 1 shows a small child "wearing" the aforesaid device. Items Nos. 3 and 4 are shown enlarged in Fig. 2. These items represent the heart of this device.

They contain a temperature sensor located on the rear side of the device, interfaced by a microchip able to detect, via an analogue-to-digital converter, the body temperature of the subject.

The temperature detected appears on a liquid-crystal display and is constantly configured according to a threshold set by the user by means of the selector shown in Fig. 2.

When the threshold set is overstepped, a piezoelectric buzzer is activated.

The device is supplied by a battery, and the ON/OFF switch acts directly on the power supply circuit.

In order to prevent mere pressure exerted by parts of the subject's body from altering the operation of the device, access may be gained to the threshold selector only by using the tip of a pencil or similar object. The switch for turning on the device is set into the front of the device.

Items Nos. 5 and 6 constitute the system that allows the child to "wear" this device.

It is a harness made of an elasticated, ecological material and is hooked to the main device, as shown by items No. 7. This harness will be adjustale according to the age of the child.

It must also have a closing mechanism, represented in the above figure by item No. 8.

## Claims

1. Device for preventing convulsive attacks.

2. Device according to claim 1, characterized by the fact that its body includes a temperature sensor, which is able to detect the body temperature of the subject via an analogue-to-digital converter, a liquid-crystal display, a threshold selector, and activation of a piezoelectric buzzer.

3. Elastic harness in ecological material.
